# EUROPEAN PATENT SPECIFICATION

(11) **EP 1 297 828 B1**
(45) Date of publication and mention of the grant of the patent: **03.12.2008**
(21) Application number: 01951330.8
(22) Date of filing: 07.06.2001
(51) Int. Cl.: A61K 9/48, A61K 47/36

(54) **ORAL FORMULATIONS FOR LOCALIZED COLONIC RELEASE AND THE METHOD OF PREPARATION THEREOF**
ORALE FORMULIERUNGEN ZUR LOKALEN FREISETZUNG IM KOLON UND METHODE DEREN ZUBEREITUNG
COMPOSITION PHARMACEUTIQUE A ADMINISTRATION ORALE LIBEREE AU NIVEAU COLIQUE ET SON PROCEDE DE PREPARATION

(30) Priority: 07.06.2000 CN 00117989
(43) Date of publication of application: 02.04.2003
(73) Proprietor: Zhang, Hao, Nanjing City, Jiangsu 210009 (CN)
(72) Inventor: ZHANG, Junshou, Nanjing City, Jiangsu 210009 (CN)
(74) Representative: Stuttard, Garry Philip
(86) International application number: PCT/CN2001/000919
(87) International publication number: WO 2002/039982

(56) References cited:
- WO-A-00/28974
- WO-A-92/00732
- WO-A-99/18938
- WO-A1-00/28974
- WO-A1-99/18938
- CN-A- 1 208 343
- US-A1- 5 840 332
- RUBINSTEIN A ET AL: "IN VITRO AND IN VIVO ANALYSIS OF COLON SPECIFICITY OF CALCIUM PECTINATE FORMULATIONS" EUROPEAN JOURNAL OF PHARMACEUTICS AND BIOPHARMACEUTICS, ELSEVIER SCIENCE PUBLISHERS B.V., AMSTERDAM, NL, vol. 41, no. 5, 1 October 1995 (1995-10-01), pages 291-295, XP000674395 ISSN: 0939-6411
- ASHFORD M ET AL: "STUDIES ON PECTIN FORMULATIONS FOR COLONIC DRUG DELIVERY" JOURNAL OF CONTROLLED RELEASE, ELSEVIER, AMSTERDAM, NL, vol. 30, no. 3, 1 July 1994 (1994-07-01), pages 225-232, XP000453419 ISSN: 0168-3659
- PORNSAK SRIAMORNSAK: "EFFECT OF CALCIUM CONCENTRATION, HARDENING AGENT AND DRYING CONDITION ON RELEASE CHARACTERISTICS OF ORAL PROTEINS FROM CALCIUM PECTINATE GEL BEADS" EUROPEAN JOURNAL OF PHARMACEUTICAL SCIENCES, ELSEVIER, AMSTERDAM, NL, vol. 8, no. 3, 1999, pages 221-227, XP002962494 ISSN: 0928-0987
- RUBINSTEIN A ET AL: "The rationale for peptide drug delivery to the colon and the potential of polymeric carriers as effective tools" JOURNAL OF CONTROLLED RELEASE, ELSEVIER, AMSTERDAM, NL, vol. 46, no. 1-2, 5 May 1997 (1997-05-05), pages 59-73, XP004096312 ISSN: 0168-3659
- DAWN A ADKIN ET AL: "The Use of Scintigraphy to Provide 'Proof of Concept' for Novel Polysaccharide Preparations Designed for Colonic Drug Delivery" PHARMACEUTICAL RESEARCH, KLUWER ACADEMIC PUBLISHERS-PLENUM PUBLISHERS, NE, vol. 14, no. 1, 1 January 1997 (1997-01-01), pages 103-107, XP019370600 ISSN: 1573-904X
- PORNSAK SRIAMORNSAK EUR. J. PHARM. SCI. vol. 8, no. 3, 1999, pages 221 - 227, XP002962494
- DATABASE CAPLUS [Online] ASHFORD MARIANNE ET AL.: 'Studies on pectin formulations for colinic', XP002960302 Database accession no. 121:117505 & J. CONTROLLED RELEASE vol. 30, no. 3, 1994, pages 225 - 232

## Description

### FIELD OF THE INVENTION

The invention relates to an oral preparation for specific delivery in colon and preparation method for thereof.

### BACKGROUND OF THE INVENTION

Many drugs cannot be delivered well via gastrointestinal tract due to a variety of reasons. For example, the diseases or conditions in the colon of human body, such as colitis, ulcerative colitis or colon cancer, etc., are located at the distal portion of the alimentary canal, so when administrated orally, a drug is general absorbed in the upper side of small intestine, the concentration of drug at colon is quite low or even close to zero. The furthest effect of administration by enema or suppository can arrive to rectum or sigmoid colon, rather than ascending colon and transverse colon, and said administration is not convenient. In U.S. Patent, 5,840,332, E. Itzbak Lerner and et al. disclose a gastrointestinal (including colon) drug delivery system, in which calcium pectinate is used as a water-insoluble material, wherein said calcium pectinate contains 2-4% of calcium by weight. However, said patent gives only the embodiments of using said calcium pectinate to prepare sodium salicylate tablets and the results of delivery of said tablets in the stomach, and the embodiment of capsules comprising said calcium pectinate is not involved.

WO 99/18938 A, RUBINSTEIN A ET AL, JOURNAL OF PHARMACEUTICS AND BIOPHARMACEUTICS, ELSEVIER SCIENCE PUBLISHERS B.V., AMSTERDAM, NL, vol. 41, no. 5, 1 October 1995 (1995-10-01), pages 291-295, ASHFORD M ET AL, JOURNAL OF CONTROLLED RELEASE, ELSEVIER, AMSTERDAM, NL, vol. 30, no. 3, 1 July 1994 (1994-07-01), pages 225-232, and PORNSAK SRIAMORNSAK, EUROPEAN JOURNAL OF PHARMACEUTICAL SCIENCES, ELSEVIER, AMSTERDAM, NL, vol. 8, no. 3, 1999, pages 221-227, disclose gastrointestinal drug delivery systems comprising metallic salts of pectin having up to 4%(w/w) of calcium.

Hence, it is very important to study and develop a capsule formulation for specific delivery in colon.

### OBJECT OF THE INVENTION

The object of the present invention is to study and develop an oral preparation for specific delivery in colon.

### SUMMARY OF THE INVENTION

After thorough studies carried out by the inventor, it is surprisingly found that the capsule preparation comprising a metallic salt of pectin containing 5-12 wt% of metal has excellent effects of specific delivery of drug in colon. The present invention is completed based on the above finding.

According to the present invention, the oral preparation is tablet or capsule.

The first aspect of the present invention relates to a capsule for specific delivery in colon, which comprises a drug, a metallic salt of pectin, and other pharmaceutically acceptable additives or excipients.

The other aspect of the present invention relates to a tablet preparation for specific delivery in colon, which comprises a drug containing core, and particles of metal pectinate having 5-12% (w/w) of metal.

The other aspect of the present invention relates to methods for preparation of the capsule preparation of the present invention, which comprises:
a) Mixing a drug and pharmaceutically acceptable excipients or additives, and processing to obtain pellets;
b) Coating the pellets of step a) with a in-situ formed metallic salt of pectin having 5-12% (w/w) of metal;
c) Spray coating a ethanol solution of acrylic polymer on coated pellets of step b), and then encapsulating into gelatin capsules; or
   a') Firstly preparing capsule shells of metallic salt of pectin having 5-12% (w/w) of metal;
   b') Encapsulating a drug into the capsule shells prepared in step a').

The other aspect of the present invention also relates to the method for preparation of the tablet preparation for specific delivery in colon, which comprises: preparing coated tablets with a tablet core and particles of metallic salt of pectin having 5-12% (w/w) of metal by dry tabletting proecess; or press molding a mixture of a drug and a metallic salt of pectin having 5-12% (w/w) of metal to obtain tablet cores, and then spray coating said tablet cores with a coating agent such as ethanol solution of acrylic polymer.

According to the invention, the drug for capsule preparation is selected from a group consisting of anti-inflammatory agents, antimicrobial agents, anticancer agents, immunodepressants, angiomyocardiacs, anti-colitis agents, a variety of Chinese Traditional Herbs and etc.

According to the invention, the term "anti-inflammatory agents" comprises steroids or non-steroidic anti-inflammatory agents, such as indometacine, hydrocortisone and etc.

According to the invention, the term "immunodepressants" comprises cyclosporin and etc.

According to the invention, the term "angiomyocardiacs" comprises nifedipinc and etc.

According to the invention, the term "anticancer agents" comprises 5-fluorouracil and etc.

According to the invention, the term "anti-colitis agent" comprises 5-amino-salicylic acid and etc.

According to the invention, the term "Chinese Traditional Herbs" comprises Chinese Traditional Herbs for treating or preventing a variety of diseases or conditions, such as Chinese Traditional Herbs having the function of immuno-adjusting or antibiotic or anti-inflammatory functions, Chinese Traditional Herbs for treating diabetes, and Chinese Traditional Herbs for treating hypertension.

### DETAILED DESCRIPTION OF THE PREFERRED EMBODIMENTS

The present invention relates to a capsule preparation for specific delivery in colon, which comprises a drug, a metallic salt of pectin having 5-12% (w/w) of metal, pharmaceutically acceptable carriers or excipients, and normal capsule shells.

The present invention also relates to a capsule preparation for specific delivery in colon, which contains a drug, pharmaceutically acceptable carriers or excipients, and capsule shells containing a metallic salt of pectin having 5-12% (w/w) of metal.

More specifically, the capsule preparation for specific delivery in colon in the present invention comprises: pellets comprising a drug and pharmaceutically acceptable excipients or additives, a coating of metallic salt of pectin having 5-12% (w/w) of metal and a coating of acrylic polymer, and normal capsule shells.

According to the present invention, the preferable capsule preparation for specific delivery in colon comprises pellets containing 5-amino-salicylic acid, a coating of calcium pectinate having 5-12% (w/w) of calcium, a coating of acrylic polymer and normal capsule shells.

According to the present invention, the preferable capsule preparation for specific delivery in colon comprises 5-amino-salicylic acid, pharmaceutically acceptable carriers or excipients, and capsule shells of calcium pectinate having 5-12% (w/w) of calcium and water content of 6-10% (w/w).

According to the present invention, the oral tablet preparation for specific delivery in colon comprises drug-containing tablet cores, and particles of metallic salt of pectin having 5-12% (w/w) of metal.

More specifically, the oral tablet preparation for specific delivery in coloh of the present invention comprises core tablets having 5-amino-salicylic acid, and particles of calcium pectinate having 5-12% of calcium.

According to the present invention, the oral tablet preparation comprises tablet cores which comprise a metallic salt of pectin (such as calcium pectinate) having 5-12% (w/w) of metal (calcium) and a drug (such as 5-amino-salicylic acid)), and a coating agent.

According to the present invention, the methods for preparation of the capsule preparation for specific delivery in colon comprises:
a) Mixing a drug and pharmaceutically acceptable excipients or additives, and processing to obtain pellets;
b) Coating the pellets of step a) with a in-situ formed metallic salt of pectin having 5-12% (w/w) of metal;
c) Spray coating a ethanol solution of acrylic polymer on coated pellets of step b), and then encapsulating into gelatin capsules; or
   a') Firstly preparing capsule shells of metallic salt of pectin having 5-12% (w/w) of metal;
   b') Encapsulating a drug into the capsule shells prepared in a').

The present invention also relates to the methods for preparation of the oral tablet preparation for specific delivery in colon, which comprises: preparing coated tablets with a tablet core and particles of metallic salt of pectin having 5-12% (w/w) of metal by dry tabletting proecess; or press molding a mixture of a drug and a metallic salt of pectin having 5-12% (w/w) of metal to obtain tablet cores, and then spray coating said tablet cores with a coating agent such as ethanol solution of acrylic polymer.

The present invention also relates to the capsule preparation for specific delivery in colon prepared by the above methods.

According to the present invention, the drug of the capsule preparation in the present invention is preferably 5-amino-salicylic acid.

According to the present invention, the metallic salt of pectin is selected from calcium pectinate, ferric pectinate or zinc pectinate, preferably calcium pectinate.

According to the present invention, the pharmaceutically acceptable carriers or excipients of step a) is selected from at least one of the group consisting of starch, microcrystalline cellulose, saccharose, lactose, mannitol, water, ethanol-water, ethanol solution of polyvinylpyrrolidone, slurry of hydroxypropylmethylcellulose, lowly substituted hydroxylpropylcellulose, sodium carboxymethyl-starch and etc., and the pellets of step a) can be prepared by centrifuging granulation, pressing method or high-speed stirring granulation. In the step b), the method for coating the pellets with metallic salt of pectin comprises: spray coating a solution of metallic salt (such as 1-8% (w/w) ethanol solution of CaCl₂) on the pellets while rolling, dipping the pellets with CaCl₂ into a water solution of pectin for 15-60 minutes, and then dipping into a hot ethanol solution of metallic salt such as CaCl₂. In the step c), the acrylic polymer is selected from Eudragit L or Eudragit S.

According to the methods of the present invention, the capsule shells of metallic salt of pectin having 5-12% (w/w) of metal and 6-10 wt% of water in step a') are prepared by:
i) Mixing lower methoxy-pectin with a cross-linking agent selected from a group consisting of formaldehyde, glutaraldehyde, sodium alginate, gelatin, arabia gum, peach gum, methylcellulose, ethylcellulose, polyvinylpyrrolidone, hydroxypropylmethylcellulose, chitosan or acrylic resin, a plasticizer selected from propylene glycol, glycerin, diethyl phthalate, dibutyl sebate, tributyl citrate or castor oil, and water, holding and degassing at 50°C to form a glue liquid;
ii) Coating clean mold rods with a liquid paraffin as lubricant, then dipping in the glue liquid of step i) for 15 seconds to 1 minute, and drawing out from the glue liquid;
iii) Dipping the solidified mold rods of step ii) into an 0.1-10% (w/w) ethanol solution of a metallic salt such as CaCl₂ to calcify, and holding at 40-80 °C for 10 minutes to 5 hours;
iv) Drying the solidified mold rods of step iii) by air blowing at 30-60 °C and 30-40% humidity until the water content is 6-10 wt%.
v) When necessary, dipping the mold rods of step iv) into a 1-10% (w/v) solution of polyvinylpyrrolidone for a moment, drawing out and drying with hot air, then dipping into a 1-10% (w/v) solution of acrylic resin for a moment, drawing out and drying with hot air, demolding and cutting according to the needed size to obtain said capsule shells.

According to the method for preparation of the oral tablet preparation, the core tablets are prepared by: mixing a drug such as 5-amino-salicylic acid with one or more pharmaceutically acceptable carriers selected from a group consisting of microcrystalline cellulose, starch, lactose and etc. to obtain a mixture, adding starch slurry or ethanol solution of polyvinylpyrrolidone or 50% ethanol to obtain a soft stuff, sieving and granulating, drying and shaping obtained particles, adding 1% disintegrating agent such as magnesium stearate into the particles and mixing, tabletting to obtain said tablet cores. The particles of calcium pectinate are prepared by: treating commercially obtained pectin by ion exchange method or acidifying-alcohol method to obtain pure pectin; treating purified pectin with acids and alkalis to obtain a de-esterified pectin, i.e., a lower methoxy-pectin; formulating said lower methoxy-pectin to obtain a water solution; and adding into an ethanol solution with a certain concentration of calcium ion to obtain a calcium pectinate precipitate; and washing obtained precipitate, drying, smashing and granulating with starch slurry, gelatin slurry, peach gum slurry, HPMC slurry, MC slurry or EC slurry to obtain said calcium pectinate particles.

The following examples further describe the present invention, but the examples are not intended to limit the invention in any sense.

### EXAMPLE 1

### CAPSULES FOR SPECIFIC DELIVERY OF 5-AMINO-SALICYLIC ACID IN COLON

**Table 1**

| Component | Amount |
|---|---|
| 5-Amino-salicylic acid | 125 g |
| Starch | 25 g |
| Dextrin | 3.6 g |
| 3% Ethanol solution of calcium chloride | Proper amount |

The above components were processed by the steps a) to c) of aforementioned method to obtain capsules for specific delivery of 5-amino-salicylic acid in colon.

### EXAMPLE 2

### PREPARATION OF CAPSULE SHELLS HAVING CALCIUM PECTINATE

**Table 2**

| Component | Amount |
|---|---|
| 15% water solution of lower methoxy-pectin (LMP) | 100 ml |
| 5% Ethanol-water (7:3) solution of CaCl₂ | 100 ml |
| 5% Ethanol solution of PVP | 100 ml |
| 8% Alcohol solution of type-II acrylic resin | 100 ml |

Clean mold rods were coated with liquid paraffin, then dipped into the 15% LMP solution for 30 seconds, drawn out and dipped into 5% ethanol solution of CaCl₂ and calcified (60 °C) for 1 hour again, dried by air blowing at 35 °C and RH 35% and dipped again into 5% ethanol solution of PVP for 2 minutes, drawn out and dried by air blowing at 35 °C and RH 35% to nearly dry, dipped into 8% alcohol solution of type-II acrylic resin for 1 minute, drawn out and dried to obtain said calcium pectinate capsule shells.

### EXAMPLE 3

### PREPARATION OF CAPSULE SHELLS HAVING CALCIUM PECTINATE

**Table 3**

| Component | Amount |
|---|---|
| 15% water solution of lower methoxy-pectin (LMP) | 100 ml |
| Arabia gum or peach gum | 2 g |
| 5% Ethanol-water (7:3) solution of CaCl₂ | 100 ml |
| 5% Ethanol solution of PVP | 100 ml |
| 8% Alcohol solution of type-II acrylic resin | 100 ml |

Arabia gum or peach gum was dissolved into the LMP solution, or the LMP and arabia gum or peach gum was independently dissolved into a proper amount of water and then mixted to uniform, and then propylene glycol was added to obtain a glue liquid. The residual processes are identical with those disclosed in example 2.

### EXAMPLE 4

### PREPARATION OF CAPSULE SHELLS HAVING CALCIUM PECTINATE

**Table 4**

| Component | Amount |
|---|---|
| 15% water solution of lower methoxy-pectin (LMP) | 100 ml |
| Gelatin or methylcellulose or hydroxypropylmethyl cellulose or sodium alginate | 2 g |
| Glycerin | 2 g |
| 5% Ethanol-water (7:3) solution of CaCl₂ | 100 ml |
| 5% Ethanol solution of PVP | 100 ml |
| 8% Alcohol solution of type-II acrylic resin | 100 ml |

The capsule shells were prepared by the method of example 3.

### EXAMPLE 5

### PREPARATION OF CAPSULE SHELLS HAVING CALCIUM PECTINATE

**Table 5**

| Component | Amount |
|---|---|
| 15% Water solution of lower methoxy-pectin (LMP) | 100 ml |
| 5% Ethanol solution of ethylcellulose | 100 ml |
| Diethyl phthalate or dibutyl sebate | 1.5 g |
| 5% Ethanol-water (7:3) solution of CaCl₂ | 100 ml |
| 5% Ethanol solution of PVP | 100 ml |

Clean mold rods were coated with liquid paraffin, then dipped into the 15% LMP solution for 1 minute, drawn out and dipped into the 5% CaCl₂ ethanol (70%) solution, calcified at 60°C for 1 hour, dried by air blowing at 35°C and RH35% to nearly dry, and then dipped into an ethanol solution of ethylcelullose having diethyl phthalate or dibutyl sebate for 30 seconds, drawn out and dried to obtain calcium pectinate capsule shells.

### EXAMPLE 6

### PREPARATION OF CALCIUM PECTINATE

**Table 6**

| Component | Amount |
|---|---|
| Pectin | 15 g |
| Ammonia water | 250 ml |
| Ethanol | 400 ml |
| CaCl₂ | 80 g |
| Distilled water | As required |

Pectin was mixed and suspended in a mixture liquid of ethanol and ammonia water, and stirred for 6 hours and filtered. The filter residue was washed with ethanol, dried at 60 °C and sieved with 100 mesh sieve, dissolved into 2000 ml distilled water by a 60°C waterbath, and then 1000 ml of 5% CaCl₂ solution was added. After filtration, the so obtained filter residue was washed with distilled water until no Cl could be detected, then dried at 80 °C and smashed to obtain a 100 mesh of fine powder. The amount of calcium is 9.43 wt% as detected by the atomic absorption spectrometry.

### EXAMPLE 7

### CAPSULES FOR SPECIFIC DELIVERY OF 5-AMINO-SALICYLIC ACID IN COLON

**Table 7**

| Component | Amount |
|---|---|
| 5-Amino-salicylic acid | 125 g |
| L-HPC | 5 g |
| Talc | 3.9 g/1000 capsules |

Preparation process: mixing each of the above components and encapsulating into the capsule shells as prepared in examples 2 to 5.

### EXAMPLE 8

### TABLETS FOR SPECIFIC DELIVERY OF 5-AMINO-SALICYLIC ACID IN COLON

**Table 8**

| Component | Amount |
|---|---|
| 5-Amino-salicylic acid | 125 g |
| Calcium pectinate | 200 g |
| 5% Ethanol solution of ethylcellulose | As required |
| HPMC | 50 g |
| Talc | 3%/1000 tablets |

Preparation process: uniformly mixing 5-amino-salicylic acid, calcium pectinate and HPMC, adding 5% ethanol solution of ethylcellulose to obtain a soft stuff, sieving and granulating to obtain particles, drying at 50 °C, shaping obtained particles, adding talc, tabletting and coating with 8% alcohol solution of type-III acrylic resin.

### EXAMPLE 9

### TABLETS FOR SPECIFIC DELIVERY OF 5-AMINO-SALICYLIC ACID IN COLON

**Table 9**

| Component | Amount |
|---|---|
| 5-Amino-salicylic acid | 125 g |
| Calcium pectinate | 375 g |
| Lactose | 25 g |
| HPMC | 100 g |
| 10% Starch slurry | As required |
| 10% Alcohol solution of ethylcellulose | 100 g |
| Talc | 30% |
| Magnesium stearate | As required |

Preparation process: mixing 5-amino-salicylic acid with lactose, adding 10% starch slurry to obtain a soft stuff, sieving and granulating to obtain particles, drying at 50°C, shaping obtained particles, adding talc and tabletting (7 mm punch die) to obtain tablet-cores; mixing calcium pectinate with HPMC, adding 10% alcohol solution of ethylcellulose to obtain another soft stuff, sieving and granulating, drying at 60°C, shaping obtained particles, adding talc and mixed to uniform, and tabletting with said tablet-cores by a φ11 mm punch die to obtain dry-pressed coated tablets.

### EXAMPLE 10

### TABLETS (1000 TABLETS) FOR SPECIFIC DELIVERY OF 5-AMINO-SALICYLIC ACID (5-ASA) IN COLON

**Table 10**

| Component | Amount |
|---|---|
| 5-Amino-salicylic acid (5-ASA) | 125 g |
| Hydroxypropylmethylcellulose (HPMC) | 25 g |
| Carbopol | 10 g |
| Ethylcellulose (EC) | 10 g |
| Calcium pectinate (Cap) | 200 g |
| 95% Ethanol | As required |

The coating agent liquid is a 6% acrylic resin of enteric coating agent.

Preparation process: weighing 5-ASA, HPMC, Carbopl, EC and Cap powders according to the amounts as given in table 10 and mixing, adding 80% ethanol to obtain a soft stuff, sieving with 22 mesh sieve and granulating to obtain particles, drying at 50°C, shaping obtained particles, adding an amount of talc powder as required and tabletting by a φ7 mm punch die to obtain tablet-cores, then coating with said coating agent.

### EXAMPLE 11

### DRY-PRESSED COATED CORE TABLETS (1000 TABLETS) FOR SPECIFIC DELIVERY OF 5-AMINO-SALICYLIC ACID (5-ASA) IN COLON

**Table 11-1**

| Component | Amount |
|---|---|
| 5-Amino-salicylic acid (5-ASA) | 125 g |
| Sodium carboxymethyl-starch | 10 g |
| 4% HPMC liquid | As required |
| Talc powder | 4.0 g |

Preparation process: mixing 5-ASA and sodium carboxymethyl-starch, adding 4% HPMC liquid to obtain a soft stuff, sieving with 20 mesh sieve and granulating to obtain particles, drying at 50 °C, shaping obtained particles, adding talc powder and tabletting by a φ7 mm punch die with shallow concave to obtain core tablets.

**Table 11-2**

| Component | Amount |
|---|---|
| Calcium pectinate | 200 g |
| HPMC | 30 g |
| Arabia gum | 20 g |
| 10% alcohol solution of ethylcellulose | As required |
| Talc | 7.5 g |

Calcium pectinate, HPMC and Arabia gum were uniformly mixed, then 10% alcohol solution of ethylcellulose was added to obtain a soft stuff. The soft stuff was sieved with 18 mesh sieve and granulated to obtain particles. The particles were shaped and mixed with talc, and then tabletted with the said core tablets by a φ11 mm punch die with shallow concave (each of said core tablet uses about 0.3g of calcium pectinate particles).

### EXAMPLE 12

### IN VITRO DEGREE OF RELEASE OF 5-AMINO-SALICYLIC ACID IN TABLETS OF EXAMPL 8

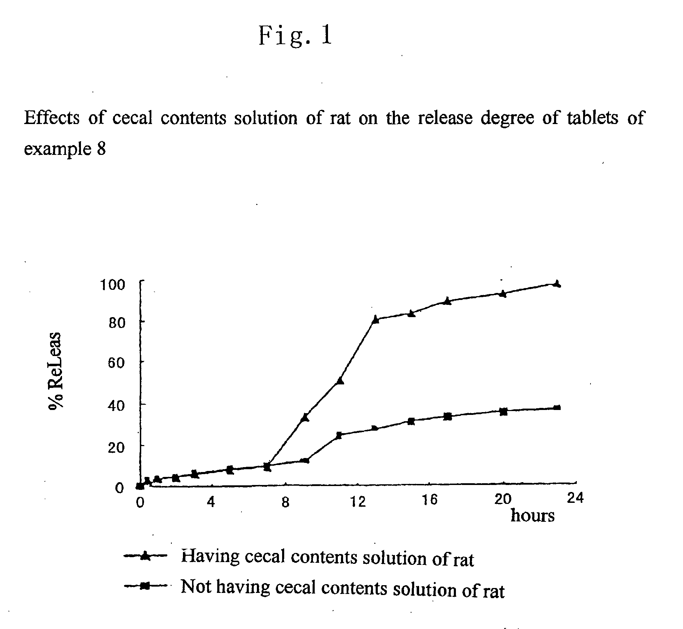

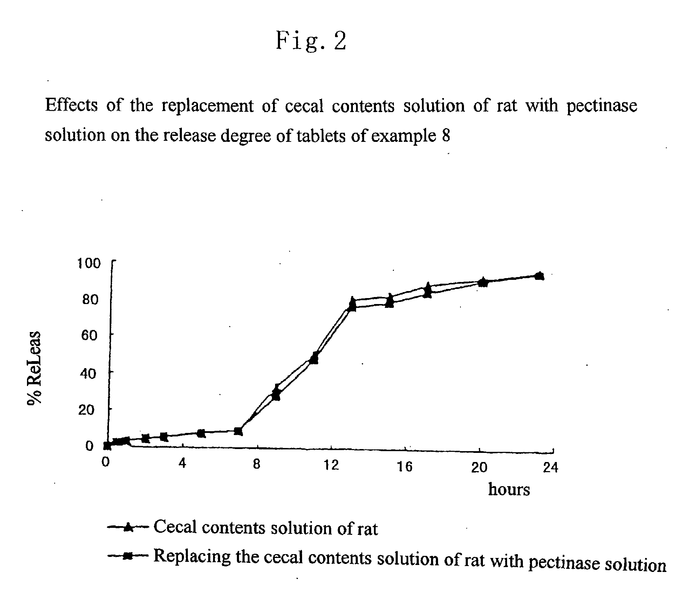

The titled degree is determined by the second method for determining the diffusion degree of drug from tablets in the Chinese Pharmacopoeia, 95 Edition. The media is a water solution with pH 1.2 during 0-2 hours, a phosphoric acid buffer with pH 6.8 during 3-7 hours, and a pectinase solution or a solution of cecal contents of rat during 8-24 hours. The speed of rotation is 50 rpm, and the temperature is 37±0.5 °C. The results are set forth in Fig. 1 and Fig. 2.

According to Fig. 1 and Fig. 2, the amount of released drug from tablets of example 8 during 2 hours in media of pH 1.2 is 1%^{±}, and during 5 hours in media of pH 6.8 is 10%^{±}, i.e., the amount of released drug during 0-7 hours is not more than 15%, while during 8-23 hour, the amount of released drug in either pectase solution or solution of cecal contents of rat is > 85%, which means that said tablets meet the requirements for specific delivery of drug in colon.

### EXAMPLE 13

### IN VIVO VERIFICATION OF TARGETING PROPERTIES OF ^{99m}TcO₄ TABLETS

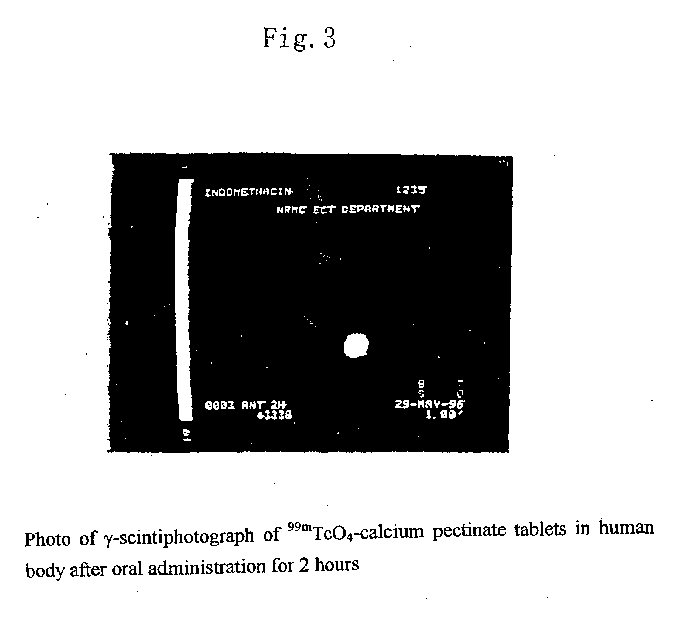

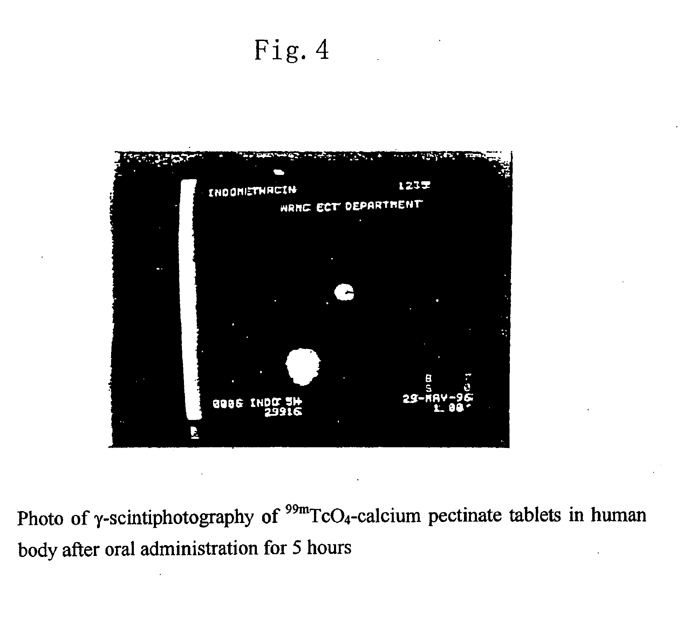

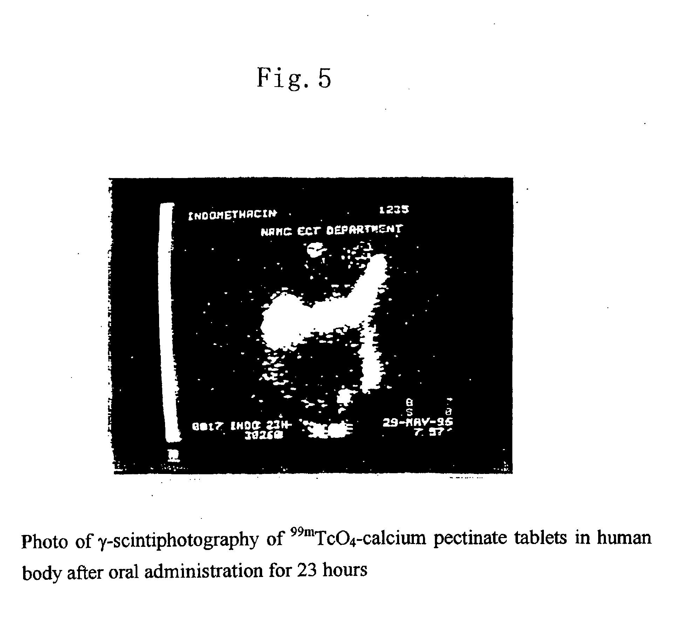

Similarly with the method for preparation of tablets of example 8, the ^{99m}TcO₄-Cap tablets were obtained by replacing 5-amino-salicylic acid with the water solution of ^{99m}TcO₄. Two healthy volunteers orally took said ^{99m}TcO₄-Cap tablets with empty stomach, and the human body images at still position were obtained by γ-scintiphotograph after oral administration for 2, 5 and 23 hours respectively. The results showed that the ^{99m}TcO₄-Cap tablets maintained their initial shape in stomach and small intestine of human body so that the ^{99m}TcO₄ was prevented from release and can safely pass through the stomach and small intestine, while it can be released completely in colon. It could be seen from the photo of scintiphotograph of ^{99m}TcO₄-Cap tablets obtained in vivo after oral administration for 23 hours that ^{99m}TcO₄ was almost diffusively distributed in whole colon. Therefore, it can be believed that prepared calcium pectinate does possess properties of targeting delivery of drug in colon, and prepared ^{99m}TcO₄-Cap tablets meet the requirement of targeting delivery of drug in vivo of human body. The photos of γ-scintiphotograph of ^{99m}TcO₄-Cap tablets obtained after oral administration for 2, 5 and 23 hours can be seen in Figures 3, 4 and 5.

### Annotation:

The position marking was not carried out in the photo of γ-scintiphotograph of 2 hours, while the position marking substances are shown as small bright spots on the upside of the photos of γ-scintiphotograph of 5 and 23 hour. The ^{99m}TcO₄-Cap tablets maintained their initial shape and showed no notable change in stomach after oral administration for 2 hours; expanded in a small degree and substantially maintained their tablet shape in small intestine after 5 hours; and completely disintegrated after 23 hours, the radioactive ^{99m}TcO₄ was diffusively distributed in whole ascending colon, transverse colon and descending colon.

## Claims

1. A capsule for specific delivery in colon, comprising a drug, a metallic salt of pectin having 5-12% (w/w) of metal, and other pharmaceutically acceptable carriers or excipients.

2. The capsule according to claim 1, wherein the metallic salt of pectin is selected from calcium pectinate, ferric pectinate or zinc pectinate

3. The capsule according to claim 1 or 2, wherein the metallic salt of pectin is calcium pectinate.

4. The capsule according to claim 1 or 2, comprising a drug of 5-amino-salicylic acid, pharmaceutically acceptable carriers or excipients, and capsule shells of calcium pectinate having 5-12% (w/w) of calcium and 6-10% (w/w) of water.

5. A tablet for specific delivery in colon, comprising tablet-cores having a drug, and particles of metallic salt of pectin having 5-10% of metal.

6. The tablet according to claim 5, wherein the drug is 5-amino-salicylic acid, and the metallic salt of pectin is calcium pectinate.

7. A tablet for specific delivery in colon, comprising core tablets comprising a drug, a metallic salt of pectin having 5-12% (w/w) of metal, other pharmaceutically acceptable carriers or excipients, and coating agents.

8. A method for preparation of the capsule according to any one of claims 1-4, comprising:
a) Mixing a drug and pharmaceutically acceptable excipients or additives, and processing to obtain pellets;
b) Coating the pellets of step a) with a in-situ formed metallic salt of pectin having 5-12% (w/w) of metal;
c) Spray coating a ethanol solution of acrylic polymer on coated pellets of step b), and then encapsulating into gelatin capsules; or
a') Firstly preparing capsule shells of metallic salt of pectin having 5-12% (w/w) of metal;
b') Encapsulating a drug into the capsule shells prepared in a').

9. A capsule shell suitable for a capsule preparation for specific delivery in colon, **characterized in that** said capsule shell comprising a metallic salt of pectin having 5-12% (w/w) of metal and 6-10% of water.

10. A method for preparation of the capsule shell according to claim 9, comprising:
i) Mixing lower methoxy-pectin with a cross-linking agent selected from a group consisting of formaldehyde, glutaraldehyde, sodium alginate, gelatin, arabia gum, peach gum, methylcellulose, ethylcellulose, polyvinylpyrrolidone, hydroxypropylmethyl cellulose, chitosan or acrylic resin, a plasticizer selected from propylene glycol, glycerin, diethyl phthalate, dibutyl sebate, tributyl citrate or castor oil, and water, holding and degassing at 50 °C to form a glue liquid;
ii) Coating clean mold rods with a liquid paraffin as lubricant, then dipping in the glue liquid of step i) for 15 seconds to 1 minute, and drawing out from the glue liquid;
iii) Dipping the mold rods of step ii) into an 0.1-10% (w/w) ethanol solution of a metallic salt such as CaCl₂ to calcify, and holding at 40-80°C for 10 minutes to 5 hours;
iv) Drying the solidified mold rods of step iii) by air blowing at 30-60 °C and 30-40% humidity until the water content is 6-10 wt%.
v) When needed, dipping the mold rods of step iv) into a 1-10% (w/v) solution of polyvinylpyrrolidone for a moment, drawing out and drying with hot air, then dipping into a 1-10% (w/v) solution of acrylic resin for a moment, drawing out and drying with hot air, demolding and cutting according to the needed size to obtain said capsule shells.

## Patentansprüche

1. Kapsel zur spezifischen Abgabe im Kolon, umfassend einen Wirkstoff, ein Metallsalz von Pectin mit 5-12% (G/G) Metall und andere pharmazeutisch verträgliche Träger oder Exzipientien.

2. Kapsel gemäß Anspruch 1, wobei das Metallsalz von Pectin ausgewählt ist aus Calciumpectinat, Eisen(III)-pectinat und Zinkpectinat.

3. Kapsel gemäß Anspruch 1 oder 2, wobei das Metallsalz von Pectin Calciumpectinat ist.

4. Kapsel gemäß Anspruch 1 oder 2, umfassend einen Wirkstoff von 5-Aminosalicylsäure, pharmazeutisch verträgliche Träger oder Exzipientien und Kapselhüllen aus Calciumpectinat mit 5-12% (G/G) Calcium und 6-10% (G/G) Wasser.

5. Tablette zur spezifischen Abgabe im Kolon, umfassend Tablettenkerne mit einem Wirkstoff und Partikel aus Metallsalz von Pectin mit 5-10% Metall.

6. Tablette gemäß Anspruch 5, wobei der Wirkstoff 5-Aminosalicylsäure ist und das Metallsalz von Pectin Calciumpectinat ist.

7. Tablette zur spezifischen Abgabe im Kolon, umfassend Kerntabletten, umfassend einen Wirkstoff, ein Metallsalz von Pectin mit 5-12% (G/G) Metall, andere pharmazeutisch verträgliche Träger oder Exzipientien, und Beschichtungsmittel.

8. Verfahren zur Herstellung der Kapsel gemäß einem der Ansprüche 1 bis 4, umfassend:
a) Mischen eines Wirkstoffs und pharmazeutisch verträglicher Exzipientien oder Additive und Verarbeiten, wobei Pellets erhalten werden;
b) Beschichten der Pellets aus Stufe a) mit einem in situ gebildeten Metallsalz von Pectin mit 5-12% (G/G) Metall;
c) Sprühbeschichten der beschichteten Pellets aus Stufe b) mit einer Ethanollösung eines Acrylpolymers und anschließendes Verkapseln in Gelatinekapseln; oder
a') zuerst Herstellen von Kapselhüllen aus Metallsalz von Pectin mit 5-12% (G/G) Metall;
b') Verkapseln eines Wirkstoffs in den in a') hergestellten Kapselhüllen.

9. Kapselhülle, die für eine Kapselpräparation zur spezifischen Abgabe im Kolon geeignet ist, **dadurch gekennzeichnet, dass** die Kapselhülle ein Metallsalz von Pectin mit 5-12% (G/G) Metall und 6-10% Wasser umfasst.

10. Verfahren zur Herstellung der Kapselhülle gemäß Anspruch 9, umfassend:
i) Mischen von Niedermethoxypectin mit einem Vernetzungsmittel, ausgewählt aus einer Gruppe, bestehend aus Formaldehyd, Glutaraldehyd, Natriumalginat, Gelatine, Gummi arabicum, Pfirsichgummi, Methylcellulose, Ethylcellulose, Polyvinylpyrrolidon, Hydroxypropylmethylcellulose, Chitosan und Acrylharz, einem Weichmacher, ausgewählt aus Propylenglycol, Glycerin, Diethylphthalat, Dibutylsebacat, Tributylcitrat und Ricinusöl, und Wasser, Halten und Entgasen bei 50°C, wobei eine Klebstoffflüssigkeit gebildet wird;
ii) Beschichten sauberer Formstäbe mit einem flüssigen Paraffin als Schmiermittel, anschließend Eintauchen in die Klebstoffflüssigkeit aus Stufe i) für 15 Sekunden bis 1 Minute und Herausziehen aus der Klebstoffflüssigkeit;
iii) Eintauchen der Formstäbe aus Stufe ii) in eine 0,1-10% (G/G) Ethanollösung eines Metallsalzes, wie CaCl₂, zur Calcifizierung und Halten bei 40-80°C für 10 Minuten bis 5 Stunden;
iv) Trocknen der verfestigten Formstäbe aus Stufe iii) mittels Aufblasen von Luft mit 30-60°C und 30-40% Feuchtigkeit, bis der Wassergehalt 6-10 Gew.-% beträgt;
v) falls erforderlich, Eintauchen der Formstäbe aus Stufe iv) in eine 1-10% (G/V) Lösung von Polyvinylpyrrolidon für einen Moment, Herausziehen und Trocknen mit heißer Luft, anschließend Eintauchen in eine 1-10% (G/V) Lösung von Acrylharz für einen Moment, Herausziehen und Trocknen mit heißer Luft, Ablösen von der Form und Zurechtschneiden gemäß der erforderlichen Größe, wobei die Kapselhüllen erhalten werden.

## Revendications

1. Capsule pour une délivrance spécifique dans le côlon, comprenant un médicament, un sel métallique de pectine comportant 5 à 12 % en poids de métal, et d'autres supports ou excipients pharmaceutiquement acceptables.

2. Capsule selon la revendication 1, dans laquelle le sel métallique de pectine est choisi parmi le pectinate de calcium, le pectinate ferrique ou le pectinate de zinc.

3. Capsule selon la revendication 1 ou 2, dans laquelle le sel métallique de pectine est le pectinate de calcium.

4. Capsule selon la revendication 1 ou 2, comprenant un médicament d'acide 5-amino-salicylique, des supports ou des excipients pharmaceutiquement acceptables, et des tuniques de capsules de pectinate de calcium comportant 5 à 12 % en poids de calcium et 6 à 10 % en poids d'eau.

5. Comprimé pour une délivrance spécifique dans le côlon, comprenant des noyaux de comprimés renfermant un médicament et des particules de sel métallique de pectine comportant 5 à 10 % de métal.

6. Comprimé selon la revendication 5, dans lequel le médicament est l'acide 5-amino-salicylique et le sel métallique de pectine est le pectinate de calcium.

7. Comprimé pour une délivrance spécifique dans le côlon, comprenant des noyaux de comprimés renfermant un médicament, un sel métallique de pectine comportant 5 à 12 % en poids de métal, d'autres supports ou excipients pharmaceutiquement acceptables et des agents d'enrobage.

8. Procédé de préparation de la capsule selon l'une quelconque des revendications 1 à 4, comprenant :
a) le mélange d'un médicament et d'excipients ou d'additifs pharmaceutiquement acceptables et le traitement pour obtenir des pellets ;
b) l'enrobage des pellets de l'étape a) avec un sel métallique de pectine comportant 5 à 12 % en poids de métal formé *in situ* ;
c) l'enrobage par pulvérisation d'une solution éthanolique de polymère acrylique sur les pellets enrobés de l'étape b), et ensuite l'encapsulation dans des capsules de gélatine ; ou
a') la préparation en premier des tuniques des capsules de sel métallique de pectine comportant 5 à 12 % en poids de métal ;
b') l'encapsulation d'un médicament dans les tuniques des capsules préparées dans a').

9. Tunique de capsule appropriée pour une préparation de capsule pour une délivrance spécifique dans le côlon, **caractérisée en ce que** ladite tunique de capsule comprenant un sel métallique de pectine comportant 5 à 12 % en poids de métal et 6 à 10 % d'eau.

10. Procédé de préparation de la tunique de capsule selon la revendication 9, comprenant :
i) le mélange de méthoxy inférieur-pectine avec un agent de réticulation choisi dans un groupe constitué de formaldéhyde, glutaraldéhyde, alginate de sodium, gélatine, gomme arabique, gomme de pêche, méthylcellulose, éthylcellulose, polyvinylpyrrolidone, hydroxypropylméthyl-cellulose, chitosane ou une autre résine acrylique, un plastifiant choisi parmi le propylène glycol, la glycérine, le phtalate de diéthyle, le sébate de dibutyle, le citrate de tributyle ou l'huile de ricin et l'eau, le maintien et le dégazage à 50°C pour former un liquide collant ;
ii) l'enrobage de baguettes de moulage propres avec une paraffine liquide en tant que lubrifiant, puis le trempage dans le liquide collant de l'étape i) pendant 15 secondes à 1 minute, et l'extirpation hors du liquide collant ;
iii) le trempage des baguettes de moulage de l'étape ii) dans une solution éthanolique à 0,1 à 10 % en poids d'un sel métallique tel que CaCl₂ pour calcifier, et le maintien à 40 à 80°C pendant 10 minutes à 5 heures ;
iv) le séchage des baguettes de moulage solidifiées de l'étape iii) par soufflage d'air à 30 à 60°C et 30 à 40 % d'humidité jusqu'à ce que la teneur en eau soit de 6 à 10 % en poids ;
v) lorsque c'est nécessaire, le trempage des baguettes de moulage de l'étape iv) dans une solution à 1 à 10 % (p/v) de polyvinylpyrrolidone pendant un moment, l'extirpation et le séchage avec de l'air très chaud, puis le trempage dans une solution à 1 à 10 % (p/v) de résine acrylique pendant un moment, l'extirpation et le séchage avec de l'air très chaud, le démoulage et le découpage selon la taille nécessaire pour obtenir lesdites tuniques de capsules.
